# EUROPÄISCHE PATENTSCHRIFT

(11) **EP 1 430 015 B1**
(45) Veröffentlichungstag und Bekanntmachung des Hinweises auf die Patenterteilung: **01.08.2007**
(21) Anmeldenummer: 02772303.0
(22) Anmeldetag: 14.09.2002
(51) Int. Cl.: C07C 67/08, C07C 69/75, C07C 67/303

(54) **VERFAHREN ZUR HERSTELLUNG VON ALICYCLISCHEN POLYCARBONSÄUREESTERN AUS PARTIALESTERN AROMATISCHER POLYCARBONSÄUREN**
METHOD FOR THE PRODUCTION OF ALICYCLIC POLYCARBOXYLIC ACID ESTERS FROM PARTIAL ESTERS OF AROMATIC POLYCARBOXYLIC ACIDS
PROCEDE DE PRODUCTION D'ESTERS D'ACIDES POLYCARBOXYLIQUES ALICYCLIQUES A PARTIR D'ESTERS PARTIELS D'ACIDES POLYCARBOXYLIQUES AROMATIQUES

(30) Priorität: 27.09.2001 DE 10147776
(43) Veröffentlichungstag der Anmeldung: 23.06.2004
(73) Patentinhaber: Oxeno Olefinchemie GmbH, 45772 Marl (DE)
(72) Erfinder: BÜSCHKEN, Wilfried, 45721 Haltern (DE); GRASS, Michael, 45721 Haltern am See (DE); KAIZIK, Alfred, 45772 Marl (DE); MASCHMEYER, Dietrich, 45657 Recklinghausen (DE); NIERLICH, Franz, 45768 Marl (DE); TUCHLENSKI, Axel, 45478 Mülheim (DE)
(74) Vertreter: Hirsch, Hans-Ludwig
(86) Internationale Anmeldenummer: PCT/EP2002/010332
(87) Internationale Veröffentlichungsnummer: WO 2003/029179

(56) Entgegenhaltungen:
- EP-A- 0 005 737

## Beschreibung

Die Erfindung betrifft ein Verfahren zur Herstellung von Vollestern von alicyclischen Polycarbonsäuren aus Partialestern von aromatischen Polycarbonsäuren (durch Hydrierung und anschließende Veresterung).

Cycloaliphatische Polycarbonsäureester, wie beispielsweise die Ester der Cyclohexan-1,2-dicarbonsäure, werden als Schmierölkomponente und als Hilfsmittel bei der Metallverarbeitung eingesetzt. Weiterhin finden sie als Weichmacher für Polyolefine Verwendung.

Für die Weichmachung von PVC werden häufig Ester der Phthalsäure, wie beispielweise Dibutyl- , Dioctyl-, Dinonyl- oder Didecylester, verwendet. Da diese Phthalate zunehmend als gesundheitsschädlich angesehen werden, ist damit zu rechnen, dass deren Einsatz in Kunststoffen eingeschränkt werden könnte. Cycloaliphatische Polycarbonsäureester, von denen einige in der Literatur als Weichmacher für Kunststoffe beschrieben sind, könnten dann als Ersatzstoffe, wenn auch mit einem etwas anderem anwendungstechnischen Profil, zur Verfügung stehen.

Alicyclische Polycarbonsäureester können durch Veresterung der entsprechenden alicyclischen Polycarbonsäuren, deren Anhydride oder Partialester gewonnen werden. Meistens stehen diese Verbindungen nicht preiswert zur Verfügung, weil sie durch mehrstufige Synthesen hergestellt werden müssen.

Beispielsweise könnenl,2-Cyclohexandicarbonsäureester durch Diels-Alder-Reaktion von Butadien mit Maleinsäureanhydrid, Fumaraten oder Maleinaten und Hydrierung der olefinischen Doppelbindung des Cyclohexenderivats erhalten werden. Wird Maleinsäureanhydrid als Ausgangsstoff verwendet, schließt die Synthese zusätzlich eine Veresterung ein.

In vielen Fällen werden alicyclische Polycarbonsäureester durch Kernhydrierung der entsprechenden aromatischen Polycarbonsäureester hergestellt.

In US 5 286 898 und US 5 319 129 werden Verfahren beschrieben, mit dem Dimethylterephthalat an geträgerten Pd-Katalysatoren, die mit Ni, Pt und/oder Ru versetzt sind, bei Temperaturen größer oder gleich 140 °C und einem Druck zwischen 50 und 170 bar zum entsprechenden Hexahydrodimethylterephthalat hydriert werden können. In DE 28 23 165 ist ein Verfahren zur Hydrierung von aromatischen Carbonsäureestern an geträgerten Ni-, Ru-, Rh- und/oder Pd-Katalysatoren zu den entsprechenden cycloaliphatischen Carbonsäureestern bei 70 bis 250 °C und 30 bis 200 bar offenbart. In US 3 027 398 wird die Hydrierung von Dimethylterephthalat an geträgerten Ru-Katalysatoren bei 110 bis 140 °C und 35 bis 105 bar beschrieben.

DE 197 56 913 bzw WO 99/32427 offenbaren ein Verfahren zur Hydrierung von Benzolpolycarbonsäureestern zu den entsprechenden cycloaliphatischen Verbindungen. Dabei werden Trägerkatalysatoren eingesetzt, die Ru alleine oder zusammen mit mindestens einem. Metall der I, VII oder VIII Nebengruppe des Periodensystems enthalten und 50 % Makroporen aufweisen.

Die zur Kernhydrierung eingesetzten aromatischen Carbonsäureester werden meistens durch Veresterung der entsprechenden Carbonsäuren oder deren Anhydriden hergestellt, wobei die entsprechenden Partialester im Reaktionsgemisch als Zwischenprodukt entstehen. Dabei können bis zur vollständigen Veresterung längere Reaktionszeiten bzw. drastischere Reaktionsbedingungen notwendig sein.

Die in der o. g. Literatur beschriebenen Verfahren zur Herstellung von cycloaliphatischen Polycarbonsäureestern gehen somit von der folgenden Reaktionssequenz aus:
1. Herstellung eines aromatischen Polycarbonsäureesters, wobei in der Regel zunächst ein Partialester gebildet wird, der zum Vollester umgesetzt wird
2. Reinigung und Aufarbeitung des so erhaltenen Polycarbonsäureesters zum Reinprodukt,
3. Hydrierung des aromatischen Polycarbonsäureesters zum entsprechenden cycloaliphatischen Polycarbonsäureester
4. Optional: Reinigung und Aufarbeitung des so erhaltenen cycloaliphatischen Polycarbonsäureesters zum Reinprodukt.

Es sind auch Verfahrensvarianten denkbar, bei denen ungereinigter Rohester hydriert wird. Diese Verfahren haben jedoch u. a. den Nachteil, dass die Aktivität des Hydrierkontakts durch Ablagerung des Veresterungskatalysators verringert wird, wodurch ein häufiger Katalysatorwechsel erforderlich wird und somit die Wirtschaftlichkeit abnimmt.

Da die Veresterungsreaktion in der Regel homogen katalysiert ist, muss das Rohprodukt der Veresterung vor dessen Hydrierung vom Katalysator, Nebenprodukten und Alkohol befreit, d. h. aufgearbeitet werden. Diese Aufarbeitung ist aufwendig, da die gängigen Verfahren zwei Reinigungsstufen, wodurch zweimal Ausbeuteverluste entstehen können, aufweisen.

Es bestand somit die Aufgabe, ein einfacheres und wirtschaftlicheres Verfahren zur Herstellung von alicyclischen Polycarbonsäureestern bereitzustellen.

Überraschenderweise ist es möglich, durch Hydrierung eines aromatischen Partialesters einer aromatischen Polycarbonsäure oder eines Gemisches, das einen oder mehrere Partialester von einer oder mehreren aromatischen Polycarbonsäure enthält, mit anschließender Veresterung der so erhaltenen cycloaliphatischen Polycarbonsäurepartialester in einfacher und wirtschaftlicher Weise zu cycloaliphatischen Polycarbonsäureestern zu gelangen.

Gegenstand der vorliegenden Erfindung ist daher ein Verfahren zur Herstellung von cycloaliphatischen Polycarbonsäureestern, wobei
a) ein Partialester der entsprechenden aromatischen Polycarbonsäure oder des entsprechenden aromatischen Polycarbonsäureanhydrids hydriert, und
b) der so erhaltene cycloaliphatische Partialester mit einem Alkohol zu den entsprechenden Vollester umgesetzt wird.

Optional können die Vollester durch Filtration, Wasserdampfdestillation oder strippen mit Wasserdampf gereinigt werden.

Im erfindungsgemäßen Verfahren können als Edukte Reinstoffe, z. B. isomerenreine Verbindungen bezüglich der Esterseitenkette als auch Gemische von Isomeren oder auch Gemische von verschiedenen Estern, sowohl bezüglich der Kettenlänge der Estergruppe und des aromatischen Systems eingesetzt werden.

Isomerengemische bezüglich der Esterseitenkette entstehen z. B. bei der Herstellung von Phthalsäureestern des Isononanols, welches ein C₉-Isomerengemisch ist. Es ist ebenso möglich, ein Gemisch aus C₈ und C₉-Phthalsäurepartialestern und/oder ein Isomerengemisch von 1,2-und 1,4-Phthalsäurepartialestern einzusetzen. Partialester der Phthalsäure sind deren Monoester mit einer verbliebenen Carbonsäurefunktion.

In einer besonderen Ausführungsform des erfindungsgemäßen Verfahrens wird zunächst aus einer aromatischen Polycarbonsäure bzw. dem entsprechenden Anhydrid und einem Alkohol bzw. Alkoholgemisch ein Partialester hergestellt. Verfahren zur Veresterung von Carbonsäuren bzw. deren Anhydriden sind dem Fachmann bekannt. Bei der Veresterung von Polycarbonsäuren, insbesondere deren Anhydriden, entstehen zunächst Partialester, d. h. die Veresterungsreaktion verläuft nicht bis zum vollständig veresterten Polycarbonsäureester. So wird z. B. bei der Veresterungsreaktion von Phthalsäureanhydrid mit Alkoholen zunächst autokatalytisch bei erhöhter Temperatur der entsprechende Monoester gebildet. Wird jedoch der entsprechende Diester gewünscht, so ist es zweckmäßig, die zweite Veresterungsstufe bei höherer Temperatur und/oder Zugabe eines Katalysators durchzuführen.

Unter Partialestern wird in der vorliegenden Erfindung eine Verbindung verstanden, die neben mindestens einer Esterfunktion noch mindestens eine freie Carbonsäure- bzw. Anhydridfunktion beinhaltet.

Ein Vorteil des erfindungsgemäßen Verfahrens liegt darin, dass in beiden Reaktionsschritten Einfluss auf das cis/trans-Verhältnis der alicyclischen Polycarbonsäurederivate genommen werden kann, sodass Produkte mit unterschiedlichen trans-Gehalten gewonnen werden können. Nach dem erfindungsgemäßen Verfahren können Produkte mit einem höheren trans-Gehalt als bei der direkten Hydrierung des entsprechenden aromatischen Polycarbonsäureesters erhalten werden. Cis- und trans-Verbindungen weisen unterschiedliche anwendungstechnische Eigenschaften auf. Der Einsatz von trans-reichen Produkten als Weichmacher für Kunststoffe, empfiehlt sich, wenn beispielsweise eine geringere Flüchtigkeit des Weichmachers gefordert wird.

Weiterhin ist es vorteilft, dass zur Herstellung der alicyclischen Polycarbonsäureester nach dem erfindungsgemäßen Verfahren nur eine Reinigungsstufe, die aus mehreren Teilschritten bestehen kann, notwendig ist.

Im erfindungsgemäßen Verfahren können zur Hydrierung der Partialester in den meisten Fällen die gleichen Katalysatoren eingesetzt werden, die aus der Literatur für die Hydrierung aromatischer Vollester zu den entsprechenden cycloaliphatischen Vollestern bekannt sind.

Dies sind meistens Trägerkatalysatoren. Als Aktivmetall können sie prinzipiell alle Metalle der VIII Gruppe des Periodensystems enthalten. Vorzugsweise enthalten sie als Aktivmetalle Platin, Rhodium, Palladium, Kobalt, Nickel oder Ruthenium oder ein Gemisch aus zwei oder mehreren davon. Weitere Aktivmetalle können Elemente der ersten und siebten Nebengruppe des Periodensystems sein, wie beispielsweise Kupfer, Rhenium oder eine Kombination davon.

Die Trägermaterialien der Hydrierkatalysatoren können sein: Aktivkohle, Aluminiumoxid, Alumosilikat, Siliciumdioxid, Titandioxid, Zirkoniumdioxid, Magnesiumoxid, Zinkoxid oder deren Gemische.

Im erfindungsgemäßen Verfahren können beispielsweise Ruthenium-Aluminiumoxid-Katalysatoren der Degussa AG, Düsseldorf, mit den Typenbezeichnungen H14163 und B4168/10r eingesetzt werden.

Die im erfindungsgemäßen Verfahren eingesetzten Katalysatoren zur Hydrierung der aromatischen Partialester können durch Auftragen mindestens eines Metalls der VIII Nebengruppe des Periodensystems auf einen geeigneten Träger hergestellt werden.
Die Auftragung kann durch Tränken des Trägers in wässrigen Metallsalzlösungen, durch Aufsprühen entsprechender Metallsalzlösungen auf den Träger oder durch andere geeignete Verfahren erreicht werden. Als Salze der Metalle der VIII Nebengruppe des Periodensystems eignen sich die Nitrate, Nitrosylnitrate, Halogenide, Carbonate, Carboxylate, Acetylacetonate, Chlorokomplexe, Nitritokomplexe oder Aminkomplexe der entsprechenden Metalle.

Die mit der Metallsalzlösung beschichteten bzw. getränkten Träger werden anschließend, vorzugsweise bei Temperaturen von 100 bis 150 °C, getrocknet und wahlweise bei Temperaturen von 200 bis 600 °C, vorzugsweise 350 bis 450 °C, calciniert.

Anschließend werden die beschichteten und getrockneten und wahlweise calcinierten Träger durch Behandlung mit einem Gasstrom, der freien Wasserstoff enthält, bei Temperaturen von 30 bis 600 °C, vorzugsweise von 100 bis 450 °C aktiviert. Vorzugsweise besteht der Gasstrom aus einem Gemisch aus Wasserstoff und Stickstoff. Es kann zweckmäßig sein, den Wasserstoffgehalt im Laufe der Aktivierung zu erhöhen. Beispielsweise kann der Wasserstoffgehalt im Gasgemisch zu Beginn der Aktivierung 10 % und am Ende des Aktivierungsprozesses über 90 % betragen.

Diese Aktivierung kann gegebenenfalls im gleichen Reaktor, in dem der Partialester hydriert wird, durchgeführt werden. Optional kann die Aktivierung des Katalysators in Gegenwart einer flüssigen Phase, die über den Katalysator rieselt, vorgenommen werden.

Die Metallsalzlösung oder -lösungen werden in einer solchen Menge auf den Träger aufgebracht, dass der Gesamtgehalt an Aktivmetall, jeweils bezogen auf das Gesamtgewicht des Katalysators, 0,01 bis 30 Massen-%, vorzugsweise 0,01 bis 5 Massen-%, ganz besonders 0,05 bis 2 Massen-% beträgt.

Die Metalloberfläche auf den Katalysatoren betragen 1 bis 50 m²/g. Die Metalloberfläche wird mittels der von J.Lemaitre et al. "Characterization of Heterogeneous Catalysts", Hrsg. Francis Delanney, Marcel Dekker, New York 1984, S. 310-324, beschriebenen Chemiesorptionsverfahren bestimmt.

Die Trägermaterialien der Katalysatoren können makroporös, mesoporös oder mikroporös sein, oder haben Poren, die in unterschiedlichen Maße in die drei genannten Bereiche fallen. Ihre Oberfläche nach BET liegt zwischen 5 und 600 m²/g.

Im erfindungsgemäßen Verfahren wird die Hydrierung bevorzugt in flüssiger Phase durchgeführt. Die Hydrierung kann an suspendierten oder stückigen im Festbett angeordneten Katalysatoren kontinuierlich oder diskontinuierlich durchgeführt werden. Im erfindungsgemäßen Verfahren wird eine kontinuierliche Hydrierung an einem im Festbett angeordnetem Katalysator, bei dem sich unter Reaktionsbedingungen die Produkt/Edukfphase hauptsächlich im flüssigen Zustand befindet, bevorzugt.

Für das erfindungsgemäße Verfahren können unterschiedliche Verfahrensvarianten gewählt werden. Es kann adiabatisch, oder praktisch isotherm, d. h. mit einem Temperaturanstieg von typischerweise kleiner als 10 °C, ein- oder mehrstufig durchgeführt werden. Im letzteren Falle kann man alle Reaktoren, zweckmäßig Rohrreaktoren, adiabatisch oder praktisch isotherm sowie einen oder mehrere adiabatisch und die anderen praktisch isotherm betreiben. Weiterhin ist es möglich, die aromatischen Polyester im geraden Durchgang oder unter Produktrückführung zu hydrieren.

Das erfindungsgemäße Verfahren wird in der Flüssig/Gas-Mischphase oder Flüssigphase in Dreiphasenreaktoren im Gleichstrom durchgeführt, wobei das Hydriergas in an sich bekannter Weise im flüssigen Edukt/Produktstrom verteilt wird. Im Interesse einer gleichmäßigen Flüssigkeitsverteilung, einer verbesserten Reaktionswärmeabfuhr und einer hohen Raum-ZeitAusbeute werden die Reaktoren vorzugsweise mit hohen Flüssigkeitsbelastungen von 15 bis 120, insbesondere von 25 bis 80 m³ pro m² Querschnitt des leeren Reaktors und Stunde betrieben. Wird ein Reaktor im geraden Durchgang betrieben, so kann die spezifische Katalysatorbelastung (LHSV) Werte zwischen 0,1 und 10 h⁻¹ annehmen.

Die Hydrierung kann in Ab- oder vorzugsweise in Anwesenheit eines Lösungsmittels durchgeführt werden. Als Lösemittel können alle Flüssigkeiten eingesetzt werden, die mit dem Edukt und Produkt eine homogene Lösung bilden, sich unter Hydrierbedingungen inert verhalten und sich leicht vom Produkt abtrennen lassen. Das Lösemittel kann auch ein Gemisch mehrerer Stoffe sein und gegebenenfalls Wasser enthalten.

Beispielsweise können folgende Stoffe als Lösemittel eingesetzt werden:
Geradkettige oder cyclische Ether, wie beispielsweise Tetrahydrofuran oder Dioxan, sowie aliphatische Alkohole, in denen der Alkylrest 1 bis 13 Kohlenstoffatome aufweist.

Bevorzugt verwendbare Alkohole sind beispielsweise Isopropanol, n-Butanol, Isobutanol, n-Pentanol, 2-Ethylhexanol, Nonanole, technische Nonanolgemische, Decanol, technische Decanolgemische.

Bei Einsatz von Alkohol als Lösemittel kann es zweckmäßig sein, denjenigen Alkohol oder dasjenige Alkoholgemisch zu verwenden, das bei der Verseifung des Produkts entstehen würde (z. B. Isononanol als Lösungsmittel bei der Hydrierung von Phthalsäuremonoisononylester). Dadurch wird die Nebenproduktbildung durch Umesterung ausgeschlossen. Ein weiteres bevorzugtes Lösemittel ist das Hydrierprodukt selbst.

Durch die Verwendung eines Lösemittel kann die Aromatenkonzentration im Reaktorzulauf begrenzt werden, wodurch eine bessere Temperaturkontrolle im Reaktor erreicht werden kann. Dies kann eine Minimierung von Nebenreaktionen und somit eine Erhöhung der Produktausbeute zu Folge haben. Bevorzugt liegt der Aromatengehalt im Reaktorzulauf zwischen 1 und 35 %, insbesondere zwischen 5 und 25 %. Der gewünschte Konzentrationsbereich kann bei Reaktoren, die in Schlaufenfahrweise betrieben werden, durch die Zirkulationsrate (Mengenverhältnis von rückgeführten Hydrieraustrag zu Edukt) eingestellt werden.
Die Hydrierung des erfindungsgemäßen Verfahrens wird in einem Druckbereich von 5 bis 300 bar, insbesondere von 15 bis 220 bar, ganz besonders von 50 bis 200 bar durchgeführt. Die Hydriertemperaturen liegen zwischen 50 und 200, insbesondere zwischen 80 und 160 °C.

Als Hydriergase können beliebige Wasserstoff haltige Gasgemische, die keine schädlichen Mengen an Katalysatorgiften wie beispielsweise Kohlenmonoxid oder Schwefelwasserstoff enthalten, eingesetzt werden. Die Inertgasbestandteile können beispielsweise Stickstoff oder Methan sein. Bevorzugt wird Wasserstoff in einer Reinheit von größer 95 %, insbesondere größer 98 % eingesetzt.

Die im erfindungsgemäßen Verfahren bevorzugt eingesetzten aromatischen Polycarbonsäuren bzw. deren entsprechenden Anhydride beinhalten 2,3 oder 4 Carboxylfunktionen. Beispiele für solche Verbindungen sind Phthalsäure oder Trimellitsäure.

Bevorzugt enthalten die aromatischen Polycarbonsäuren bzw. die entsprechenden Polycarbonsäureanhydride 1 bis 5 kondensierte Benzolringe oder andere annelierte Reste. Beispiele für ankondensierte Benzolringe sind Benzol-, Diphenyl-, Naphthalin-, Anthracen- oder Phenanthrensysteme.

Besonders bevorzugt werden als Partialester der aromatischen Polycarbonsäure die Monoester der 1,2-, 1,3- oder 1,4-Phthalsäure (oder deren Anhydride) oder die Mono- bzw. Di-Ester der 1,2,3-; 1,2,4- oder 1,3,5-Trimellitsäure eingesetzt.

Vorzugsweise wird ein Phthalsäuremonoisononylester oder ein Phthalsäuremonooctylester oder ein Phthalsäuremonodecylester im erfindungsgemäßen Verfahren zu den entsprechenden Cyclohexandicarbonsäureestern umgesetzt.

Im erfindungsgemäßen Verfahren können Partialester folgender aromatischer Säuren eingesetzt werden:
1,2-Naphthalindicarbonsäre, 1,3-Naphthalindicarbonsäre,1,4-Naphthalindicarbonsäre, 1,5-Naphthalindicarbonsäre, 1,6-Naphthalindicarbonsäre, 1,7-Naphthalindicarbonsäre, 1,8-Naphthalindicarbonsäure, Phthalsäure, Isophthalsäure, Terephthalsäure, Benzol-1,2,3-tricarbonsäure, Benzol-1,2,4-tricarbonsäure (Trimellitsäure), Benzol-1,3,5-tricarbonsäure (Trimesinsäure), Benzol-1,2,3,4-tetracarbonsäure, Benzol-1,2,4,5-tetracarbonsäure (Pyromellitsäure). Weiterhin können Säuren eingesetzt werden, die aus den genannten Säuren durch Substitution eines oder mehrerer am aromatischen Kern gebundenen Wasserstoffatoms(e) durch Alkyl-, Cycloalkyl- oder Alkoxyalkylgruppen entstehen. Desweiteren können Polycarbonsäuren mit einem Anthracen-, Phenanthren- oder Diphenyloxid-Grundgerüst eingesetzt werden.
Die Alkoholkomponenten der Partial- und Vollester können verzweigte oder unverzweigte Alkyl-, Cycloalkyl- sowie Alkoxyalkylgruppen mit 1 bis 25, insbesondere 3 bis 15, ganz besonders 8 bis 13 C-Atomen sein.
Es ist möglich, Alkoholgemische bzw. Partialestergemische mit verschiedenen Alkoholen einzusetzen. Gemische können sowohl Alkohole verschiedener Kettenlänge oder Isomerengemische von Alkoholen gleicher Kettenlänge sein.

Weiterhin können im erfindungsgemäßen Verfahren Gemische, die mindestens zwei Partialester enthalten, eingesetzt werden. Solche Einsatzstoffe können beispielsweise sein: Gemische, die von dreibasischen Carbonsäuren durch Teilveresterung entstehen. In diesen Gemischen können Monoalkyl- und Dialkylester nebeneinander vorliegen.

Bei der Teilveresterung einer Polycarbonsäure mit einem Alkoholgemisch fällt ein Gemisch mit mindestens zwei Partialestern an.

Darüber hinaus können Gemische von Partialestern entstehen, wenn ein Gemisch von mindestens zwei Polycarbonsäuren mit einem Alkohol oder einem Alkoholgemisch umgesetzt wird.
In der Technik werden häufig preiswerte Alkoholgemische für die Veresterung eingesetzt. Beispiele dafür sind:
C₅-Alkoholgemische, hergestellt aus linearen Butenen durch Hydroformylierung und anschließender Hydrierung;
C₅-Alkoholgemische, hergestellt aus Butengemischen, die lineare Butene und Isobuten enthalten, durch Hydroformylierung und anschließende Hydrierung
C₆-Alkoholgemische, hergestellt aus einem Penten oder aus einem Gemisch von zwei oder mehreren Pentenen, durch Hydroformylierung und anschließender Hydrierung;
C₇-Alkoholgemische, hergestellt aus Triethylen oder Dipropen oder einem Hexenisomer oder einem sonstigen Gemisch von Hexenisomeren, durch Hydroformylierung und anschließende Hydrierung;
2-Ethylhexanol (2 Isomere), hergestellt durch Aldolkondensation von n-Butyraldehyd und anschließende Hydrierung;
C₉-Alkoholgemische, hergestellt aus C₄-Olefinen durch Dimerisierung, Hydroformylierung und Hydrierung. Dabei kann von Isobuten oder von einem Gemisch linearer Butene oder von Gemischen mit linearen Butenen und Isobuten ausgegangen werden. Die C₄-Olefine können mit Hilfe unterschiedlicher Katalysatoren dimerisiert werden, wie beispielsweise Protonensäuren, Zeolithe, metallorganische Nickelverbindungen oder feste nickelhaltige Kontakte. Die Hydroformylierung der C₈-Olefingemische kann mit Hilfe von Rhodium- oder Kobaltkatalysatoren erfolgen. Es gibt daher ein Vielzahl von technischen C₉-Alkoholgemischen.
C₁₀-Alkoholgemische, hergestellt aus Tripropylen durch Hydroformylierung und anschließende Hydrierung;
2-Propylheptanol (2 Isomere), hergestellt durch Aldolkondensation von Valeraldehyd und anschließende Hydrierung;
C₁₀-Alkoholgemische, hergestellt aus einem Gemisch von mindestens zwei C₅-Aldehyden durch Aldolkondensation und anschließende Hydrierung;
C₁₃-Alkolgemische, hergestellt aus Hexaethylen, Tetrapropylen oder Tributen durch Hydroformylierung und anschließende Hydrierung.
Weitere Alkoholgemische können durch Hydroformylierung und anschließende Hydrierung aus Olefinen bzw. Olefingemischen gewonnen werden, die beispielsweise bei Fischer- Tropsch-Synthesen, bei Dehydrierungen von Kohlenwasserstoffen, Metathesereaktionen, beim Polygasverfahren oder anderen technischen Prozessen anfallen.

Darüber hinaus können auch Olefingemische mit Olefinen unterschiedlicher C-Zahlen für die Herstellung von Alkoholgemischen eingesetzt werden.

Im erfindungsgemäßen Verfahren können alle Partialestergemische, hergestellt aus aromatischen Polycarbonsäuren und den obengenannten Alkoholgemischen, eingesetzt werden. Erfindungsgemäß werden bevorzugt Ester, hergestellt aus Phthalsäure oder Phthalsäureanhydrid und einem Alkohol oder einem Gemisch isomerer Alkohole mit 1 bis 25 C-Atomen, eingesetzt.

Die Partialester können als isomerenreiner Ester oder als Gemisch von isomeren Partialestern oder als Gemisch verschiedener nicht isomerer Partialester oder bevorzugt als Reaktionsgemisch, das bei Herstellung von Partialester, gegebenenfalls nach Katalysatorabtrennung, anfällt, eingesetzt werden.
Die im erfindungsgemäßen Verfahren eingesetzten Partialester können Reinsubstanzen oder technische Gemische, z.B. aus der Veresterungsreaktion sein. Gemische dieser Art enthalten neben dem Partialester z. B. Vollester, Polycarbonsäure/Polycarbonsäureanhydrid (jeweils 0 bis 15 Mol-% bezogen auf den Partialester), Alkohol, Wasser und/oder den VeresterungsKatalysator.

Optional können aromatische Polycarbonsäuren, bzw. deren Anhydride, und Alkohole (Alkoholgemische) im vorgesehenen Verhältnis, getrennt oder zusammen, in die Hydrierungsstufe gefahren werden. Beispielsweise kann Phthalsäureanhydrid und Alkohol (Alkoholgemisch) in den äußeren Umlauf eines in Schlaufenfahrweise betriebenen Hydrierreaktors eingespeist werden. Dabei erfolgt die Partialesterbildung hauptsächlich in der äußeren Schlaufe.

Die Teilveresterung der aromatischen Polycarbonsäuren kann nach bekannten Verfahren unkatalysiert oder durch Katalyse mit Lewis- oder Brönstedtsäuren oder Metallverbindungen durchgeführt werden.

Die Herstellung von Partialestern aus Anhydriden von aromatischen Polycarbonsäuren benötigt in der Regel keinen Katalysator. Beispielweise setzt sich Phthalsäureanhydrid mit Alkoholen im Temperaturbereich von 110 bis 160 °C in sehr kurzer Zeit zu den entsprechenden Partialestern um. Die Umsetzung eines Anhydrids einer aromatischen Carbonsäure kann mit einem Alkoholüberschuss durchgeführt werden. Im Partialestergemisch können geringe Mengen an Vollester vorhanden sein. Da der Hydrieraustrag ohnehin verestert wird, ist es zweckmäßig, bei der Partialesterherstellung bereits so viel Alkohol im Überschuss einzusetzen wie für die nach der Hydrierung folgende Veresterungsstufe vorgesehen ist.

Die Hydrierausträge können neben dem oder den alicyclischen Partialester(n), Vollester und alicyclischen Polycarbonsäuren, Alkohol sowie Lösemittel, Nebenprodukte und Wasser enthalten.

Diese Gemische werden bevorzugt ohne weitere Reinigung nach an sich bekannten Verfahren zu den Vollestern umgesetzt. Diese Nachveresterung kann autokatalytisch oder katalytisch, beispielsweise mit Brönstedt- oder Lewissäuren durchgeführt werden. Ganz gleich welche Art der Katalyse gewählt wird, es entsteht immer ein temperaturabhängiges Gleichgewicht zwischen den Einsatzstoffen (Partialester, ggf. Carbonsäure und Alkohol) und den Produkten (Ester und Wasser). Um das Gleichgewicht zu Gunsten des Vollesters zu verschieben, kann ein Schleppmittel eingesetzt werden, mit dessen Hilfe das Reaktionswasser aus dem Ansatz entfernt wird. Da die zur Veresterung eingesetzten Alkohole leichter als die Polycarbonsäure, deren Partialester und deren Vollester sieden und mit Wasser eine Mischungslücke aufweisen, werden sie als Schleppmittel eingesetzt, das nach Wasserabtrennung wieder in den Prozess zurückgeführt werden kann.

Der zur Bildung des Vollesters eingesetzte Alkohol oder das Alkoholgemisch, der gleichzeitig als Schleppmittel dient, wird im Überschuss, bevorzugt 5 bis 50 %, insbesondere 10 bis 30 % der zur Bildung des Vollesters aus der Polycarbonsäure notwendigen Menge eingesetzt. Als Veresterungskatalysatoren können Säuren, wie beispielsweise Schwefelsäure, Methansulfonsäure oder p-Toluolsulfonsäure, oder Metalle bzw. deren Verbindungen eingesetzt werden. Geeignet sind z. B. Zinn, Titan, Zirconium, die als feinverteilte Metalle oder zweckmäßig in Form ihrer Salze, Oxide oder löslichen organischen Verbindungen verwendet werden. Die Metallkatalysatoren sind im Gegensatz zu Protonensäuren Hochtemperaturkatalysatoren, die ihre volle Aktivität erst bei Temperaturen oberhalb 180 °C erreichen. Sie werden jedoch bevorzugt eingesetzt, weil sie im Vergleich zu Protonenkatalyse weniger Nebenprodukte, wie beispielsweise Olefine aus dem eingesetzten Alkohol, bilden. Beispielhafte Vertreter für Metallkatalysatoren sind Zinnpulver, Zinn(II)oxid, Zinn(II)oxalat, Titansäureester wie Tetraisopropylorthotitanat oder Tetrabutylorthotitanat sowie Zirconiumester wie Tetrabutylzirconat.

Bei der vollständigen Veresterung des cycloaliphatischen Partialesters kann der gleiche Alkohol oder das gleiche Alkoholgemisch eingesetzt werden, das bei der Verseifung des Partialesters der aromatischen Polycarbonsäure entstehen würde bzw. entsteht.

In einer besonderen Ausführungsform der Erfindung wird zunächst der Partialester, z. B. gemäß der zitierten Patentliteratur hergestellt.

Anschließend kann bei der vollständigen Veresterung in Schritt b) des erfindungsgemäßen Verfahrens der gleiche Alkohol oder Alkoholgemisch eingesetzt werden, wie zur Herstellung des Partialesters verwendet wurde.

Es ist auch möglich, einen anderen Alkohol oder ein anderes Alkoholgemisch bei der Veresterung in Schritt b) des Verfahrens gemäß der Erfindung einzusetzen. So ist es z. B. möglich, einen Partialester mit einer C₈-Alkoholkomponente zu hydrieren und mit einer C₉-Alkoholkomponente zum Vollester umzusetzen.

Die Katalysatorkonzentration hängt von der Art des Katalysators ab. Bei den bevorzugt eingesetzten Titanverbindungen beträgt diese 0,005 bis 1,0 Massen-% bezogen auf das Reaktionsgemisch, insbesondere 0,01 bis 0,5 Massen-%, ganz besonders 0,01 bis 0,1 Massen-%.

Die Reaktionstemperaturen liegen bei Verwendung von Titankatalysatoren zwischen 160 °C und 270 °C, vorzugsweise bei 180 bis 250 °C. Die optimalen Temperaturen hängen von den Einsatzstoffen, Reaktionsfortschritt und der Katalysatorkonzentration ab. Sie können für jeden Einzelfall durch Versuche leicht ermittelt werden. Höhere Temperaturen erhöhen die Reaktionsgeschwindigkeiten und begünstigen Nebenreaktionen, wie beispielsweise Wasserabspaltung aus Alkoholen oder Bildung farbiger Nebenprodukte. Es ist zur Entfernung des Reaktionswassers günstig, dass der Alkohol aus dem Reaktionsgemisch abdestillieren kann. Die gewünschte Temperatur oder der gewünschte Temperaturbereich kann durch den Druck im Reaktionsgefäß eingestellt werden. Bei niedrig siedenden Alkoholen wird daher die Umsetzung bei Überdruck und bei höher siedenden Alkoholen bei vermindertem Druck durchgeführt. Beispielsweise wird bei der Umsetzung von Phthalsäureanhydrid mit einem Gemisch isomerer Nonanole in einem Temperaturbereich von 170 °C bis 250 °C im Druckbereich von 1 bar bis 10 mbar gearbeitet.

Die in die Reaktion zurückzuführende Flüssigkeitsmenge kann teilweise oder vollständig aus Alkohol bestehen, der durch Aufarbeitung des azeotropen Destillats gewonnen wird. Es ist auch möglich, die Aufarbeitung zu einem späteren Zeitpunkt durchzuführen und die entfernte Flüssigkeitsmenge ganz oder teilweise durch frischen Alkohol, d. h. aus einem im Vorratsgefäß bereitstehenden Alkohol zu ersetzen.

Die Rohestergemische, die neben dem oder den Vollester(n) Alkohol, geringe Mengen an Partialester, Katalysator oder dessen Folgeprodukte und gegebenenfalls Nebenprodukte enthalten, werden nach an sich bekannten Verfahren aufgearbeitet. Die Aufarbeitung umfasst dabei folgende Schritte: Abtrennung des überschüssigen Alkohols und ggf. Leichtsieder, optional unter Einschluss einer Wasserdampfdestillation, Neutralisation der vorhandenen Säuren, Umwandlung des Katalysators in einen leichtfiltrierbaren Rückstand, Abtrennung der Feststoffe und gegebenenfalls eine Trocknung. Dabei können je nach angewendeten Aufarbeitungsverfahren die Reihenfolge dieser Schritte verschieden sein.
Aufarbeitungsverfahren für Rohestergemische werden beispielsweise in DE 197 21 347 C2 beschrieben.

Ein weiterer Gegenstand der vorliegenden Erfindung ist die Verwendung der erfindungsgemäß hergestellten alicyclischen Polycarbonsäureestern als Weichmacher in Kunststoffen. Bevorzugte Kunststoffe sind PVC, Homo- und Copolymere auf Basis von Ethylen, Propylen, Butadien, Vinylacetat, Glycidylacrylat, Glycidylmethacrylat, Acrylaten, Acrylaten mit am Sauerstoffatom der Estergruppe gebundenen, verzweigten oder unverzweigten Alkylresten mit einem bis zehn Kohlenstoffatome(n), Styrol, Acrylnitril, Homo- oder Copolymere von cyclischen Olefinen.

Als Vertreter der obigen Gruppen seien beispielsweise folgende Kunststoffe genannt:
Polyacrylate mit gleichen oder verschiedenen Alkylresten mit 4 bis 8 C-Atomen, gebunden am Sauerstoffatom der Estergruppe, insbesondere mit dem n-Butyl-, n-Hexyl-, n-Octyl- und 2-Ethylhexylrest, Polymethacrylat, Polymethylmethacrylat, Methylacrylat-Butylacrylat-Copolymere, Methylmethacrylat-Butylmethacrylat-Copolymere, Ethylen-Vinylacetat-Copolymere, chloriertes Polyethylen, Nitrilkautschuk, Acrylnitril-Butadien-Styrol-Copolymere, Ethylen-Propylen-Copolymere, Ethylen-Propylen-Dien-Copolymere, Styrol-Acrylnitril-Copolymere, Acrylnitril-Butadien-Kautschuk, Styrol-Butadien-Elastomere, Methylmethacrylat-Styrol-Butadien-Copolymere.

Darüber hinaus können die erfindungsgemäßen alicyclischen Polycarbonsäureester zur Modifizierung von Kunststoffmischungen, beispielsweise der Mischung eines Polyolefins mit einem Polyamid, eingesetzt werden.

Gemische aus Kunststoffen und den erfindungsgemäßen oder erfindungsgemäß hergestellten alicyclischen Polycarbonsäureestern sind ebenfalls Gegenstand der vorliegenden Erfindung. Geeignete Kunststoffe sind die bereits genannten Verbindungen. Solche Gemische enthalten bevorzugt mindestens 5 Gew.-%, besonders bevorzugt 20 bis 80 Gew.-%, ganz besonders bevorzugt 30 bis 70 Gew.-% der alicyclischen Polycarbonsäureester.

Gemische aus Kunststoffen, insbesondere PVC, die einen oder mehrere der erfindungsgemäßen alicyclischen Polycarbonsäureester enthalten, können beispielsweise in folgenden Produkten enthalten sein:
Gehäuse für Elektrogeräte, wie beispielsweise Küchengeräte, Computergehäuse, Gehäuse und Bauteile von Phono- und Fernsehgeräte, Rohrleitungen, Apparaten, Kabeln, Drahtummantelungen, Isolierbändern, Fensterprofilen, im Innenausbau, im Fahrzeug- und Möbelbau, Plastisolen, in Bodenbelägen, medizinische Artikel, Lebensmittelverpackungen, Dichtungen, Folien, Verbundfolien, Schallplatten, Kunstleder, Spielzeug, Verpackungsbehälter, Klebebandfolien, Bekleidung, Beschichtungen, Fasern für Gewebe.

Weiterhin können Gemische aus Kunststoff, insbesondere PVC, die einen oder mehrere der erfindungsgemäßen alicyclischen Polycarbonsäureester enthalten, beispielsweise zur Herstellung folgender Erzeugnisse verwendet werden:
Ein Gehäuse für Elektrogeräte, eine Rohrleitung, eine Vorrichtung, ein Kabel, eine Draht-Ummantelung, ein Fensterprofil, ein Bodenbelag, ein medizinischer Artikel, ein Spielzeug, eine Lebensmittelverpackung, eine Dichtung, eine Folie, eine Verbundfolie, eine Schallplatte, Kunstleder, ein Verpackungsbehälter, eine Klebebandfolie, Bekleidung, eine Beschichtung, oder eine Faser für Gewebe.

Neben den obengenannten Anwendungen können die erfindungsgemäßen alicyclischen Polycarbonsäureester als Schmierölkomponente, als Bestandteil von Kühlflüssigkeiten und Metallbearbeitungsflüssigkeiten verwendet werden.

Die folgenden Beispiele sollen die Erfindung erläutern, ohne ihren Schutzbereich einzuschränken, der sich aus der Beschreibung und den Patentansprüchen ergibt.

### Beispiel 1: Hydrierung von Diisononylphthalat (Vestinol 9) (Vergleichsbeispiel)

In einem 600 ml-Druckreaktor wurden bei einem Druck von 200 bar und bei einer Temperatur von 120 °C 590 g Vestinol 9 in Gegenwart des Ruthenium-Katalysators B4168/10r mit reinem Wasserstoff hydriert. Nach Beendigung der Wasserstoffaufnahme wurde der Reaktor entspannt und das Produkt analysiert. Der Umsatz an Diisononylphthalat betrug danach 99,9 %: Die Ausbeute an Cyclohexan-1,2-dicarbonsäure-di(isononyl)ester lag bei 99,8 %, wobei mittels ¹H-NMR-Spektroskopie ein cis/trans-Verhältnis von 97 :3 ermittelt wurde.

| | |
|---|---|
| Meßgerät: | NMR-Spektrometer Avance DPX-360 der Firma Bruker |
| Meßfrequenz: | 360 MHz |
| Probenkopf: | QNP-Probenkopf, 5mm |
| Lösungsmittel: | CDCl₃ (Deuterierungsgrad 99,8 %) |
| Standard: | Tetramethylsilan (TMS) |
| Messtemperatur: | 303 K |
| Anzahl Scans: | 32 |
| Delay: | 1 s |
| Aquisitionszeit: | 4,4 s |
| Spektrale Breite: | 7440,5 Hz |
| Pulswinkel: | 30° |
| Pulslänge: | 3,2 µs |

Zur Aufnahme der ¹H-NMR-Spektren wurden z. B. ca. 20 mg der Probe in ca. 0,6 ml CDCl₃ (mit 1 Gew.-% TMS) gelöst. Die Spektren wurden unter den oben angegebenen Bedingungen aufgenommen und auf TMS = 0 ppm referenziert.
In den erhaltenen ¹H-NMR-Spektren liessen sich die Methinsignale der cis- und trans-Dialkylhexahydrophthalate mit den chemischen Verschiebungen von ca. 2,8 ppm bzw. 2,6 ppm unterscheiden. Dabei entsprach das zu tieferem Geld verschobene Signal (größer ppm-Wert) der cis-Verbindung. Zur Quantifizierung der Isomere wurden die Integrale von 3,0 ppm bis 2,8(2) ppm und von 2,7(2) ppm bis 2,5 ppm bestimmt, wobei die Trennung der beiden Integrale in der Mittel zwischen den Signalen erfolgte. Aus den Intensitätsverhältnissen konnte das Verhältnis der beiden isomeren Strukturen bestimmt werden.

### Beispiel 2: Synthese von Phthalsäure-mono-isononylester

444 g Phthalsäureanhydrid (3 mol) und 432 g Isononanol (3 mol) (Vorstufe von Vestinol 9) wurden in einem Rundkolben mit Innenthermometer, Rührer und aufgesetztem Rückflusskühler langsam erhitzt. Bei einer Temperatur von 117 °C begann die Monoesterbildung, was sich durch einen starken Temperaturanstieg bemerkbar machte. Sofort nach Ansteigen der Temperatur wurde die Wärmezufuhr unterbrochen. Nach etwa 10 Minuten wurde der Ansatz, der inzwischen seine Endtemperatur von etwa 150 °C erreicht hatte, abgekühlt. Die Hydrierung verläuft quantitativ.

Gaschromatographisch ließ sich eine Zusammensetzung von etwa 95 Massen-% Monoester, 3 Massen-% Diester, 0,5 Massen-% Isononanol und 1,5 Massen-% Phthalsäure ermitteln.

### Beispiel 3: Hydrierung des Phthalsäure-mono-isononylester

487 g (1,67 mol) des in Beispiel 2 hergestellten Monoestergemisches wurden ohne weitere Aufarbeitung mit 240 g (1,67 mol) Isononanol (Vorstufe von Vestinol 9) gemischt und unter Stickstoffatmosphäre in einen 1000ml-Reaktor eingefüllt. Nach Zugabe von 74 g des Ruthenium-Katalysators B4168/10r wurde bei 200 bar und 120 °C mit Wasserstoff hydriert. Nach Beendigung der Kernhydrierung der aromatischen Carbonsäurederivate wurde der Reaktor entspannt.

### Beispiel 4: Veresterung des kernhydrierten Monoesters zum Diester

Der Reaktoraustrag aus Beispiel 3 wurde in eine Standard-Veresterungsapparatur überführt, mit weiteren 120 g (0,83 mol) Isononanol und etwa 0,07 g Tetrabutyltitanat gemischt. Als Schleppmittel wurde Toluol eingesetzt und mit dessen Menge im Reaktionsgemisch die Veresterungstemperatur konstant auf 180 °C gehalten. Die Veresterung verlief unter Rückfluss des vorgelegten Alkohols und Toluols bei Normaldruck, wobei das anfallende Wasser am Wasserauskreiser abgenommen wurde. Es wurde bis zu einer Säurezahl < 0,5 mg KOH/g verestert. Der Alkohol wurde an einer Claisenbrücke bis zu einem Enddruck von 10 mbar bei dieser Temperatur abdestilliert. Danach wurde auf 80 °C abgekühlt und mit 10 %-iger wässriger Natronlauge tropfenweise neutralisiert und anschließend ca. 30 Minuten weiter gerührt.
Anschließend wurde der Ester unter einem Vakuum von 10 mbar auf 180°C aufgeheizt. Bei konstanter Temperatur wurde über das Tauchrohr destilliertes oder vollentsalztes Wasser (8 % bezogen auf die Einwaage an Rohester) zugetropft. Nach Ende der Aufarbeitung wurde die Heizung abgestellt.
Das Produkt kühlte dann unter Vakuum bis auf 80°C ab und wurde dann über eine Filternutsche mit Filterpapier und Filterhilfsmittel klar gefiltert.
Das Verhältnis von cis- und trans-1,2-Cyclohexancarbonsäurediestern wurde durch ¹H-NMR-Spektoskopie bestimmt (s.o.). Es lag bei 51 zu 49.

### Beispiel 5: Veresterung des kernhydrierten Monoesters zum Diester

Die Veresterung des Reaktionsaustrags aus Beispiel 3 wurde ähnlich wie in Beispiel 4 durchgeführt, jedoch mit dem Unterschied, dass kein Toluol zugesetzt wurde. Dadurch bedingt stieg die Veresterungstemperatur bis auf 250 °C an.

Nach der Aufarbeitung ergab die ¹H-NMR-spektroskopische Untersuchung des Produktes ein cis/trans- Verhältnis von ca. 21/79.

Beispiele 4 und 5 zeigen, dass Produkte mit unterschiedlichen trans-Gehalten der 1,2-Cyclohexandicarbonsäureester durch Variation der Veresterungsbedingungen hergestellt werden können.

Die trans-Gehalte sind bei Verwendung der gleichen Hydrierbedingungen bei der Hydrierung des Mono- und des Diesters bei dem erfindungsgemäßen Verfahren höher als bei der Hydrierung von Vestinol 9 (Vergleichsbeispiel 1).

## Patentansprüche

1. Verfahren zur Herstellung von cycloaliphatischen Polycarbonsäureestern,
**dadurch gekennzeichnet,**
**dass**
a) ein Partialester der entsprechenden aromatischen Polycarbonsäure oder des entsprechenden aromatischen Polycarbonsäureanhydrids hydriert, und
b) der so erhaltene cycloaliphatische Partialester mit einem Alkohol zum Vollester umgesetzt wird.

2. Verfahren nach Anspruch 1,
**dadurch gekennzeichnet,**
**dass** die Hydrierung des aromatischen Partialesters mit einem Katalysator, enthaltend mindestens ein Metall der 8. Nebengruppe des Periodensystems durchgeführt wird.

3. Verfahren nach einem der Ansprüche 1 oder 2,
**dadurch gekennzeichnet,**
**dass** zur vollständigen Veresterung des cycloaliphatischen Partialesters der gleiche Alkohol oder das gleiche Alkoholgemisch eingesetzt wird, das bei der Verseifung des Partialesters der aromatischen Polycarbonsäure erhalten würde.

4. Verfahren nach einem der Ansprüche 1 oder 2,
**dadurch gekennzeichnet,**
**dass** zur vollständigen Veresterung des cycloaliphatischen Partialesters ein anderer Alkohol oder ein anderes Alkoholgemisch eingesetzt wird, als bei der Verseifung des Partialesters der aromatischen Polycarbonsäure entstehen würde.

5. Verfahren nach einem der Ansprüche 1 bis 4,
**dadurch gekennzeichnet,**
**dass** die aromatischen Polycarbonsäuren oder die entsprechenden Polycarbonsäureanhydride 2, 3 oder 4 Carboxylfunktionen beinhalten.

6. Verfahren nach einem der Ansprüche 1 bis 5,
**dadurch gekennzeichnet,**
**dass** die aromatischen Polycarbonsäuren oder die entsprechenden Polycarbonsäureanhydride 1 bis 5 kondensierte Benzolringe enthalten.

7. Verfahren nach einem der Ansprüche 1 bis 6,
**dadurch gekennzeichnet,**
**dass** die Alkoholkomponenten verzweigte oder unverzweigte Alkyl-, Cycloalkyl- und/oder Alkoxyalkylgruppen mit 1 bis 15 Kohlenstoffatomen sind.

8. Verfahren nach einem der Ansprüche 1 bis 7,
**dadurch gekennzeichnet,**
**dass** die Umsetzung des cycloaliphatischen Partialesters zum Vollester unter Katalyse einer Titan- oder Zirkoniumverbindung durchgeführt wird.

9. Verfahren nach einem der Ansprüche 1 bis 8,
**dadurch gekennzeichnet,**
**dass** als Partialester einer aromatischen Polycarbonsäure ein Monoester der 1,2-, 1,3- oder 1,4-Benzoldicarbonsäure eingesetzt wird.

10. Verfahren nach einem der Ansprüche 1 bis 9,
**dadurch gekennzeichnet,**
**dass** als Partialester der aromatischen Polycarbonsäure ein Mono- oder Diester der 1,2,3-, 1,2,4- oder 1,3,5- Trimellitsäure eingesetzt wird.

## Claims

1. Process for preparing cycloaliphatic polycarboxylic esters,
**characterized in that**
a) a partial ester of the corresponding aromatic polycarboxylic acid or of the corresponding aromatic polycarboxylic anhydride is hydrogenated, and
b) the resultant cycloaliphatic partial ester is reacted with an alcohol to give the full ester.

2. Process according to Claim 1,
**characterized in that**
the hydrogenation of the aromatic partial ester is carried out using a catalyst comprising at least one metal of the 8^{th} transition group of the periodic table.

3. Process according to Claim 1 or 2,
**characterized in that**
the alcohol or alcohol mixture used for the full esterification of the cycloaliphatic partial ester is the same as that which would be obtained during saponification of the partial ester of the aromatic polycarboxylic acid.

4. Process according to Claim 1 or 2,
**characterized in that**
the alcohol or alcohol mixture used for the full esterification of the cycloaliphatic partial ester is other than that which would be produced during saponification of the partial ester of the aromatic polycarboxylic acid.

5. Process according to any of Claims 1 to 4,
**characterized in that**
the aromatic polycarboxylic acids or the corresponding polycarboxylic anhydrides contains 2, 3 or 4 carboxyl functions.

6. Process according to any of Claims 1 to 5,
**characterized in that**
the aromatic polycarboxylic acids or the corresponding polycarboxylic anhydrides contain from 1 to 5 condensed benzene rings.

7. Process according to any of Claims 1 to 6,
**characterized in that**
the alcohol components are branched or unbranched alkyl, cycloalkyl, and/or alkoxyalkyl groups having from 1 to 15 carbon atoms.

8. Process according to any of Claims 1 to 7,
**characterized in that**
the reaction of the cycloaliphatic partial ester to give the full ester is carried out with catalysis by a titanium or zirconium compound.

9. Process according to any of Claims 1 to 8,
**characterized in that**
the partial ester used of an aromatic polycarboxylic acid comprises a monoester of benzene-1,2-, 1,3-, or 1,4-dicarboxylic acid.

10. Process according to any of Claims 1 to 9,
**characterized in that**
the partial ester used of the aromatic polycarboxylic acid is a mono- or diester of 1,2,3-, 1,2-4- or 1,3,5- trimellitic acid.

## Revendications

1. Procédé de préparation d'esters d'acides polycarboxyliques cycloaliphatiques, **caractérisé en ce que**
a) on hydrogène un ester partiel de l'acide polycarboxylique aromatique correspondant ou de l'anhydride polycarboxylique aromatique correspondant, et
b) on fait réagir avec un alcool, pour obtenir l'ester complet, l'ester partiel cycloaliphatique ainsi obtenu.

2. Procédé selon la revendication 1, **caractérisé en ce que** l'hydrogénation de l'ester partiel aromatique est mise en oeuvre avec un catalyseur contenant au moins un métal du 8ème groupe secondaire du Tableau Périodique.

3. Procédé selon l'une des revendications 1 ou 2, **caractérisé en ce que**, pour l'estérification complète de l'ester partiel cycloaliphatique, on utilise le même alcool ou le même mélange d'alcools que celui que l'on obtiendrait lors de la saponification de l'ester partiel de l'acide polycarboxylique aromatique.

4. Procédé selon l'une des revendications 1 ou 2, **caractérisé en ce que**, pour l'estérification complète de l'ester partiel cycloaliphatique, on utilise un alcool ou un mélange d'alcools différent de celui qui serait produit lors de la saponification de l'ester partiel de l'acide polycarboxylique aromatique.

5. Procédé selon l'une des revendications 1 à 4, **caractérisé en ce que** les acides polycarboxyliques aromatiques ou les anhydrides polycarboxyliques correspondants contiennent des fonctions 2-, 3- ou 4-carboxyle.

6. Procédé selon l'une des revendications 1 à 5, **caractérisé en ce que** les acides polycarboxyliques aromatiques ou les anhydrides polycarboxyliques correspondants contiennent 1 à 5 noyaux benzèniques condensés.

7. Procédé selon l'une des revendications 1 à 6, **caractérisé en ce que** les composants alcool sont des groupes alkyle, cycloalkyle et/ou alcoxyalkyle à chaîne droite ou ramifiée ayant 1 à 15 atomes de carbone.

8. Procédé selon l'une des revendications 1 à 7, **caractérisé en ce que** la réaction de l'ester partiel cycloaliphatique conduisant à l'ester complet est mise en oeuvre avec catalyse par un composé du titane ou du zirconium.

9. Procédé selon l'une des revendications 1 à 8, **caractérisé en ce qu'**on utilise en tant qu'ester partiel d'un acide polycarboxylique aromatique un monoester de l'acide 1,2-, 1,3- ou 1,4-benzène-dicarboxylique.

10. Procédé selon l'une des revendications 1 à 9, **caractérisé en ce qu'**on utilise en tant qu'ester partiel de l'acide polycarboxylique aromatique un monoester ou un diester de l'acide 1,2,3-, 1,2,4- ou 1,3,5-trimellitique.
